(19) Europäisches Patentamt
European Patent Office
Office européen des brevets

(11) **EP 3 363 296 A1**

(12) **EUROPEAN PATENT APPLICATION**
published in accordance with Art. 153(4) EPC

(43) Date of publication:
**22.08.2018 Bulletin 2018/34**

(51) Int Cl.:
*A23K 20/105* (2016.01)    *A23K 50/00* (2016.01)

(21) Application number: 17806875.5

(86) International application number:
**PCT/KR2017/003077**

(22) Date of filing: **22.03.2017**

(87) International publication number:
**WO 2017/209382 (07.12.2017 Gazette 2017/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.06.2016 PCT/KR2016/005786**

(71) Applicants:
• **Yun, Kwan Sik**
  **Bucheon-si, Gyeonggi-do 14549 (KR)**

• **Kimin Inc.**
  **Seoul 06771 (KR)**

(72) Inventor: **YUN, Kwan Sik**
  **Seoul 06771 (KR)**

(74) Representative: **Modiano, Micaela Nadia et al**
  **Modiano & Partners**
  **Thierschstrasse 11**
  **80538 München (DE)**

(54) **WEIGHT GAIN PROMOTING FEED ADDITIVE, LIVESTOCK FEED COMPOSITION AND LIVESTOCK BREEDING METHOD**

(57)    The present invention relates to a weight gain promoting feed additive, which is immediately absorbed without being accumulated in the body of livestock and rapidly used as an energy source, thereby promoting the growth of livestock, a livestock feed composition and a method for culturing livestock.

[Fig. 5]

**Description**

[Technical Field]

The present invention relates to a weight gain promoting feed additive that is immediately absorbed to the body of livestock, not accumulated therein, and thus is rapidly used as an energy source, a livestock feed composition and a livestock breeding method.

[Background Art]

Feed refers to a material that supplies the organic or inorganic nutrients necessary for maintaining the life of livestock and producing milk, meat, eggs, fur or leather. Feed is a mixture of nutrients such as various energy sources, proteins, vitamins and minerals needed by livestock, a growth promoter and a vaccine.

Feed plays various roles, for example, of supplying the nutrients necessary for the survival of livestock and the production of livestock products by being consumed by livestock, reinforcing immune functions, improving the quality of livestock products, and improving the environment of livestock barns.

Particularly, increased productivity of livestock is achieved by improving the barn environment or feed efficiency, and various methods to improve feed efficiency, such as adding new ingredients to the existing feed composition, varying a mixing ratio of ingredients, and changing a feeding method, have been studied.

As an example, Korean Unexamined Patent Application Publication No. 2006-35444 relates to an animal feed and a feeding method using the same and suggests animal feed containing general animal feed and bamboo charcoal to increase a body weight or body weight gain.

Fat, which is one of the essential nutrients of livestock, has a higher energy value than other nutrients and is the most expensive energy source per unit weight. Therefore, when the utilization efficiency of fat is improved in the body, not only can the productivity of livestock be improved, but also an opportunity to reduce production costs can be provided due to reduced raw material costs of feed.

A diglyceride is a lipid composition in which fatty acids are bound to the 1 and 2 or 1 and 3 positions of glycerin by transesterification between glycerin and fatty acids, and is treated differently from a general lipid called a "triglyceride." Recently, it has been found that when a diglyceride is ingested, it has a physiological effect of not increasing a blood triglyceride level and not accumulating body fat, since, compared with a general neutral lipid, the diglyceride has the same digestion and absorption mechanisms but is structurally stable and hardly resynthesized into triglycerides after being decomposed and absorbed by a lipase. Therefore, the diglyceride has been variously used for obesity treatment and weight loss. Japanese Patent Laid-Open Publication No. 2007-512407 suggests that a lipid composition containing a large amount of a diglyceride of a conjugated linoleic acid can be used for a food or a pharmaceutical composition or as a feed additive to prevent accumulation of body fat, prevent a disease, supply nutrients, or the like. Japanese Patent Laid-Open Publication No. 1996-269478 relates to a lipid composition containing 31 wt% or more of a triglyceride having two C8 to C10 heavy-chain fatty acid residues in the molecule thereof and suggests that the lipid composition is metabolized faster than conventionally used cooking oil and accumulates a small amount of fat in the body. In addition, Japanese Patent Laid-Open Publication No. 1993-56755 relates to a livestock feed additive and a livestock feed and suggests that coccidiosis can be prevented using a heavy-chain fatty acid triglyceride having 6 to 12 carbon atoms, and that the triglyceride can inhibit the abnormal accumulation of body fat.

(Patent Document 1) Korean Unexamined Patent Application Publication No. 2006-35444
(Patent Document 2) Korean Patent No. 10-0740564
(Patent Document 3) Japanese Patent Laid-Open Publication No. 2007-512407

[Disclosure]

[Technical Problem]

The present invention is directed to providing a weight gain promoting feed additive that can be immediately used as an energy source, thus promoting growth and enhancing productivity of livestock, without being accumulated as fat when fed to livestock as a lipid source, a livestock feed composition and a livestock breeding method.

[Technical Solution]

To achieve the object of the present invention, the present invention provides a weight gain promoting feed additive, which is

a lipid composition including 50 to 70 wt% of a diglyceride, and a triglyceride, a monoglyceride, a free fatty acid or a mixture thereof as the remainder for a total of 100 wt%,

wherein the diglyceride includes 40 wt% or more of 1,3-diglyceride, and among the constitutive fatty acids of the diglyceride, fatty acids having 14 carbon atoms or less account for 70 to 90 wt% and fatty acids having 16 carbon atoms or more account for 10 to 30 wt%, and fatty acids having 14 carbon atoms or less account for 60 to 80 wt% with respect to fatty acids bound to the 1 and 3 positions among the constitutive fatty acids of the diglyceride.

The present invention also provides a livestock feed composition including the weight gain promoting feed additive and a formulated feed mixture.

The present invention also provides a livestock breeding method including supplying the livestock feed composition.

The present invention also provides a livestock feed composition prepared by substituting 40 to 60 wt% of a lipid source in a formulated feed mixture with the weight gain promoting feed additive.

The present invention also provides a livestock breeding method that includes supplying the livestock feed composition.

[Advantageous Effects]

A weight gain promoting feed additive according to the present invention can be immediately used as an energy source without being accumulated as fat during a lipid metabolism in bodies of livestock, thereby promoting growth and enhancing productivity of livestock.

[Description of Drawings]

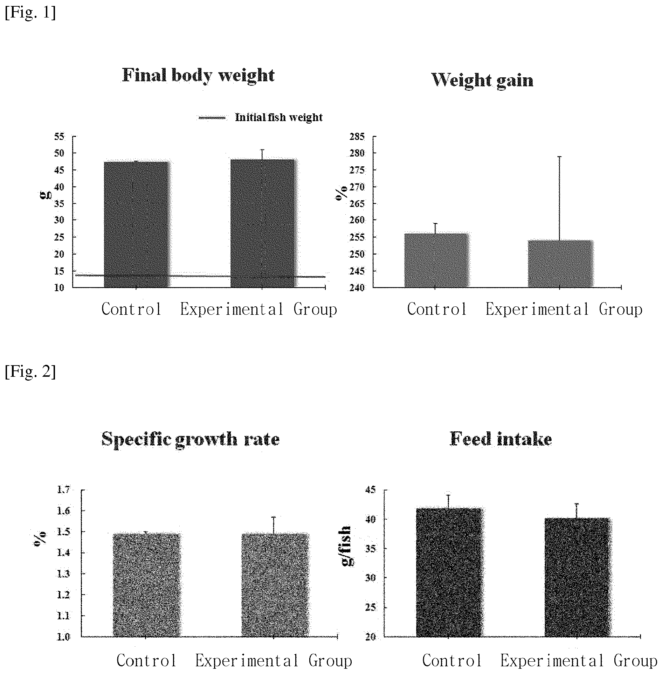

FIG. 1 is a set of graphs representing a body weight and body weight gain of flatfish determined at completion of an experiment described in Experimental Example 4, which is performed to confirm the effects of dietary supplementation of a weight gain promoting feed additive according to the present invention on culture of flatfish.

FIG. 2 is a set of graphs representing a specific growth rate and a feed intake rate of flatfish determined according to Experimental Example 4, which is performed to confirm the effects of dietary supplementation of a weight gain promoting feed additive according to the present invention on the raising of flatfish.

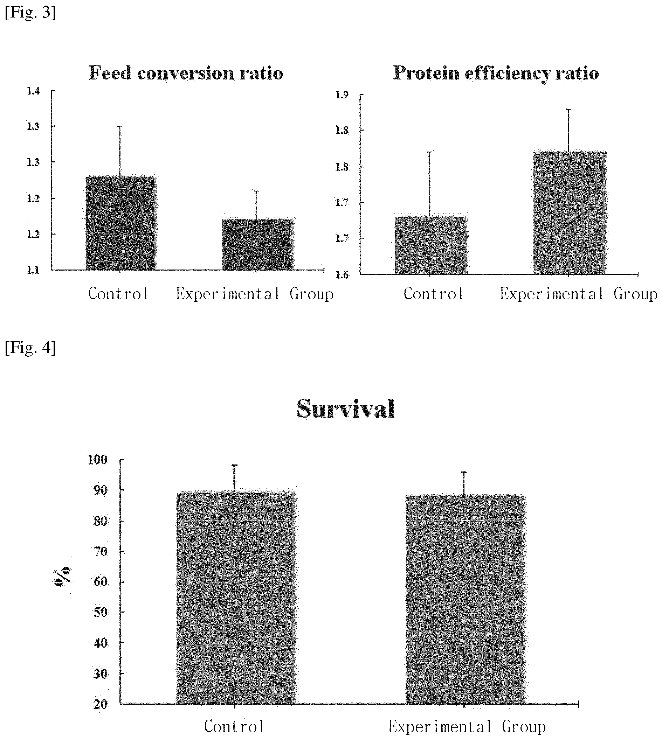

FIG. 3 is a set of graphs representing a feed conversion ratio and a protein efficiency ratio of flatfish determined according to Experimental Example 4, which is performed to confirm the effects of dietary supplementation of a weight gain promoting feed additive according to the present invention on the raising of flatfish.

FIG. 4 is a graph representing a survival rate of flatfish determined according to Experimental Example 4, which is performed to confirm the effects of dietary supplementation of a weight gain promoting feed additive according to the present invention on the raising of flatfish.

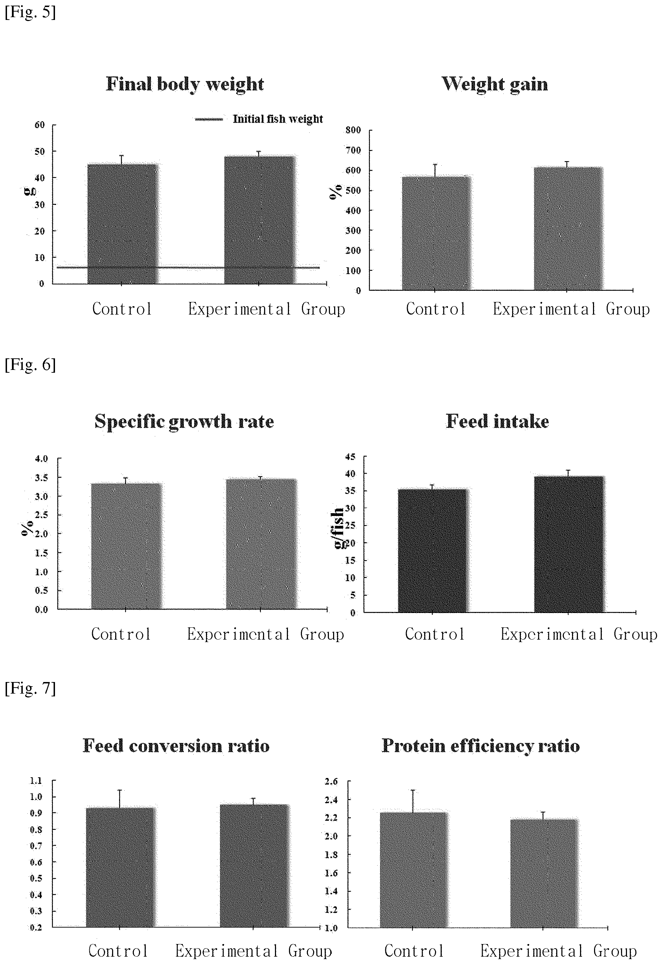

FIG. 5 is a set of graphs representing a body weight and body weight gain of flatfish determined at completion of an experiment of Experimental Example 5, which is performed to confirm the effects of dietary supplementation of a weight gain promoting feed additive according to the present invention on the raising of flatfish.

FIG. 6 is a set of graphs representing a specific growth rate and a feed intake rate of flatfish determined according to Experimental Example 5, which is performed to confirm the effects of dietary supplementation of a weight gain promoting feed additive according to the present invention on the raising of flatfish.

FIG. 7 is a set of graphs representing a feed conversion ratio and protein efficiency ratio of flatfish according to Experimental Example 5, which is performed to confirm the effects of dietary supplementation of a weight gain promoting feed additive according to the present invention on the raising of flatfish.

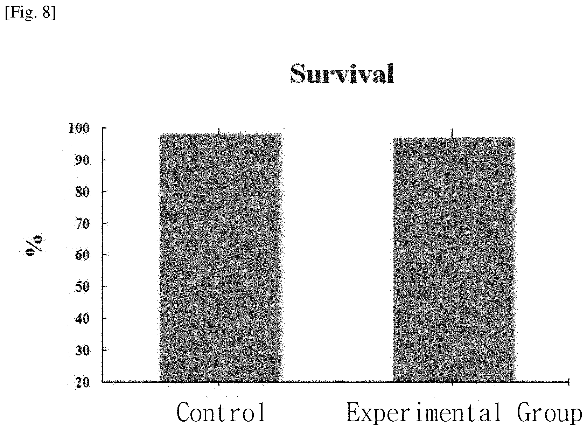

FIG. 8 is a graph representing the survival rate of flatfish determined according to Experimental Example 5, which is performed to confirm the effects of dietary supplementation of a weight gain promoting feed additive according to the present invention on the raising of flatfish.

[Modes of the Invention]

Hereinafter, the present invention will be described in further detail.

A weight gain promoting feed additive of the present invention includes 50 wt% or more of a diglyceride, wherein, among constitutive fatty acids thereof, fatty acids having 14 carbon atoms or less account for 10 to 90 wt%, and fatty acids having 14 carbon atoms or less and bound to the 1 and 3 positions account for 10 to 80 wt%.

The weight gain promoting feed additive includes a large amount of a diglyceride having a high content of fatty acids having 14 carbon atoms or less as constitutive fatty acids. Generally, a triglyceride used as a lipid source in feed is degraded into 2-monoglyceride and free fatty acid by a lipolytic enzyme in bodies of livestock, and the 2-monoglyceride is resynthesized into a triglyceride and is accumulated as fat in subcutaneous tissue. In contrast, 1,3-diglyceride is degraded into glycerin by a lipolytic enzyme and is not converted into 2-monoglyceride, which is resynthesized into a triglyceride, and the free fatty acids obtained by degradation are delivered to the liver and rapidly metabolized as an energy source. Therefore, the lipid composition for feed of the present invention is not accumulated when being supplied as a lipid source for livestock but is readily used as an energy source, thus enhancing the productivity of livestock.

In addition, the fatty acids having 14 carbon atoms or less constituting a diglyceride are degraded into free fatty acids by an enzyme in the body, easily absorbed in small intestinal cells, and transferred to the liver through the portal vein as free fatty acids, and thus are rapidly metabolized as an energy source. However, fatty acids having 16 carbon atoms or more are regenerated to a triglyceride by esterification by intestinal mucosal cells and reacted with a protein, etc. to become a lipoprotein called chylomicron, followed by being delivered to the entire body and accumulated as body fat. Unlike the fatty acids having 16 carbon atoms or more, the fatty acids having 14 carbon atoms or less do not need carnitine to be introduced into mitochondria for β-oxidation, and thus are rapidly metabolized.

Particularly, in the weight gain promoting feed additive of the present invention, a weight ratio of the fatty acids having 14 carbon atoms or less over the fatty acids having 16 carbon atoms or more is 4 to 7, among the constitutive fatty acids of a diglyceride, is 4 to 7, as obtained by increasing a content of the fatty acids having 14 carbon atoms or less, which are not accumulated when being degraded into free fatty acids, and reducing a content of the fatty acids having 16 carbon atoms or more, which are resynthesized into a triglyceride and accumulated as body fat in the body.

The diglyceride contains 1,3-diglyceride as a main component. Preferably, the diglyceride contains 40 wt% or more of the 1,3-a diglyceride. 1,2-diglyceride and 2,3-diglyceride are degraded into 2-monoglyceride and a fatty acid by a lipolytic enzyme, such as a lipase, in the body of livestock. The 2-monoglyceride and the fatty acid are used as energy sources, or the fatty acid that is not used as an energy source is used to resynthesize a glyceride or triglyceride and thus is accumulated as fat in subcutaneous tissue. On the other hand, since the 1,3-diglyceride is degraded into a glycerol and two fatty acids by a lipase in the body of livestock, when the 1,3-diglyceride is given, blood of livestock has a high fatty acid level and a low triglyceride level compared to when 1,2-diglyceride or 2,3-diglyceride is given.

Such a diglyceride is contained at 50 to 70 wt% in the weight gain promoting feed additive of the present invention. If the content is below the above-mentioned range, a content of a triglyceride, which is a cause of excessive accumulation of fat in livestock, is relatively increased. In contrast, when the content exceeds the above-mentioned range, economic feasibility may be reduced due to the need of an additional purification process, and since the content of a triglyceride required for the growth of livestock and the improvement of a meat quality is reduced, growth may be retarded or a meat quality may be degraded; therefore, the content of a diglyceride needs to be suitably selected within the above-mentioned range.

It is preferable, in a physiological aspect, that the constitutive fatty acids of a diglyceride include 70 to 90 wt% of fatty acids having 14 carbon atoms or less and 10 to 30 wt% of fatty acids having 16 carbon atoms or more so that they can be readily used as energy sources. Particularly, it is preferable that fatty acids having 14 carbon atoms or less account for 60 to 80 wt% of the fatty acids constituting the diglyceride, bound to the 1 and 3 positions, and degraded into free fatty acids by a lipase in the body of livestock.

Here, the fatty acids having 14 carbon atoms or less among the constitutive fatty acids of the diglyceride may be any type known in the art, and the present invention is not limited to a particular type of fatty acid. As a representative example, one selected from the group consisting of caprylic acid, pelargonic acid, capric acid, undecanoic acid, lauric acid, myristic acid and a mixture thereof is used. In a physiological aspect for being readily used as an energy source, it is more preferable that one selected from the group consisting of caprylic acid, capric acid, lauric acid, myristic acid and a mixture thereof is used.

As the fatty acid having 16 carbon atoms or more, one type selected from the group consisting of palmitic acid, oleic acid, linoleic acid, stearic acid and a mixture thereof is preferable, and one type selected from the group consisting of palmitic acid, stearic acid and a mixture thereof is more preferable in terms of the oxidation stability of a lipid composition and the digestion and absorption of the fatty acid.

The weight gain promoting feed additive of the present invention may be used to substitute a lipid source in a formulated feed mixture or added to a formulated feed mixture as an additive.

The livestock feed composition of the present invention includes the weight gain promoting feed additive, and based on the total weight of the formulated feed mixture, contains 0.005 to 2.5 wt% of a diglyceride, and specifically, the livestock feed composition includes 0.01 to 5.0 wt%, preferably 0.01 to 3.0 wt%, of the weight gain promoting feed additive based on the total weight of the formulated feed mixture. When a content of the weight gain promoting feed additive in the livestock formulated feed mixture is below the above-mentioned range, it is difficult to expect an effect of using the feed additive, and when the content of the feed additive exceeds the above-mentioned range, the nutritive conditions of livestock being raised may be imbalanced.

In addition, in the livestock feed composition of the present invention, 40 to 60 wt% of a lipid source of the formulated feed mixture may be substituted with the weight gain promoting feed additive. When the lipid source of the formulated feed mixture is substituted, there is no variation in quality unlike in the cases of beef tallow, fish oil and bean milk, which have been conventionally used as a lipid source, and there is an economical advantage due to low costs of use.

The livestock feed composition may be formulated to include the lipid composition of the present invention in addition to any formulated feed mixture known in the art or commercially available, and since the composition and preparation method of livestock formulated feed mixture may vary according to the type of livestock to be fed, and the present invention is not limited to a specific composition and preparation method of a livestock feed mixture. Here, the livestock may be pigs, chickens, ducks, quails, goose, pheasants, turkeys, cow, bulls, cows, horses, donkeys, sheep, goat, dogs, cats, rabbits, or various types of raised fish and shrimps.

In addition, the feed composition according to the present invention may include various types of antibiotics, probiotics, enzymes, organic acids, flavors, sweeteners, antioxidants, and other functional substances as necessary to improve the health conditions of an animal, or to obtain positive effects for productivity enhancement and production of high-quality livestock products.

The feed composition according to the present invention may be supplied during the normal period of time until the livestock reaches a weight corresponding to a purpose of raising the livestock.

The present invention provides a livestock breeding method that includes supplying the livestock feed composition to livestock.

When livestock is fed with the livestock feed composition according to the present invention, daily gain and feed efficiency are increased, resulting in substantial improvement in productivity, compared to when a conventional feed is supplied. Particularly, a blood triglyceride level is reduced, and fatty acid levels are increased in the livestock.

Hereinafter, examples and experimental examples of the present invention will be provided. However, the following examples are merely examples of the present invention and do not limit the present invention.

**Example 1: Preparation of lipid composition as feed additive**

A glyceride composition according to the present invention was obtained by preparing an ester compound having an acid value of 1 or less through esterification of fatty acid mixture (400 g) and glycerol (92 g) for 2 hours at 250 °C by adding NaOH as an alkali catalyst. The fatty acids and glyceride of the prepared glyceride composition were analyzed by the method described below, and results thereof are shown in Table 1 and Table 2 below.

1. Gas chromatography for analysis of fatty acid composition

For analysis, a sample was injected at a concentration of 25 g/l under conditions including HP-INNOWAX (Agilent, USA) as a column, helium (2.1 ml/min) as a carrier gas, an oven temperature of 150 to 260 °C, and a flame ionization detector (FID) of 275 °C.

2. Liquid chromatography for analyzing glyceride composition

As a column (liquid chromatography) for analysis of a glyceride composition, Supelcosil LC-Si (5 $\mu$m, 25 cm; Aupelco, USA) was used, and solvent A (benzene: chloroform: acetic acid = 70:30:2) and solvent B (ethyl acetate) were used as mobile-phase solvents. A sample was injected at a concentration of 1 mg/ml (in a chloroform solvent), and the analysis was carried out using an evaporation light scattering detector (ELSD) under a condition of a flow rate of 2.3 ml/min.

3. Liquid chromatography for analyzing position isomers of glyceride

As a column, ChromSpher Lipids (5 $\mu$m, 25 cm; Varian, USA) was used, and n-hexane containing 0.5% acetonitrile was used as a mobile-phase solvent, and a sample was injected at a concentration of 1 mg/ml (in a chloroform solvent). An analysis was carried out using an ELSD under a condition of a flow rate of 2.3 ml/min.

[Table 1]

| Composition | | | Content (wt%) |
|---|---|---|---|
| Free fatty acids | | | 0.6 |
| Composition of glyceride | monoglyceride | | 24.5 |
| | diglyceride | 1,3-DG | 40.5 |
| | | 1,2-DG | 13.5 |
| | triglyceride | | 20.9 |

[Table 2]

| Composition of fatty acids constituting diglyceride | Example 1 | | |
|---|---|---|---|
| | 1,3 | 1,2 | Total |
| C8:0(caprylic acid) | 8.2 | 10.2 | 8.7 |
| C10:0(capric acid) | 7.8 | 9.8 | 8.3 |
| C12:0(lauric acid) | 47.0 | 58.2 | 49.8 |
| C14:0(myristic acid) | 16.4 | 13.2 | 16.4 |
| C16:0(palmitic acid) | 9.6 | 5.6 | 8.8 |
| C18:0(stearic acid) | 11.0 | 3.0 | 8.0 |
| Fatty acids having 14 carbon atoms or less / Fatty acids having 16 carbon atoms or more | 3.9 | 10.6 | 5.0 |

**Experimental Example 1: Evaluation of lipid composition for weaning pigs**

Experimental Animal and Experiment Design

One hundred and sixty three-way crossbred [(Landrace×Yorkshire)×Duroc] weaning pigs were tested, their body weights at the initiation of the experiment were 6.84 ± 0.87kg, and the feeding experiment was performed for 6 weeks. For an experimental feed, two types of feed, namely a positive control (PC) and a negative control (100 kcal lower than PC), were prepared. In this case, each animal group was treated as follows, each treatment was repeated four times, and 5 pigs were randomly allotted for each treatment: (% is a percentage by weight (wt%))

- Positive control: feed

  - Experiment Group 1: feed+0.1 wt% of lipid composition of Example 1

  - Experiment Group 2: feed+0.2 wt% of lipid composition of Example 1

  - Experiment Group 3: feed+0.3 wt% of lipid composition of Example 1

- Negative control: 100 kcal-reduced feed

  - Comparative Group 1: 100 kcal-reduced feed+0.1 wt% of lipid composition of Example 1
  - Comparative Group 2: 100 kcal-reduced feed+0.2 wt% of lipid composition of Example 1
  - Comparative Group 3: 100 kcal-reduced feed+0.3 wt% of lipid composition of Example 1

Experimental feed, feeding and management

Pigs were free-fed with a general feed consisting mainly of corn and soybean meal and formulated in accordance with the NRC (2012) nutrient requirements as experimental feed, and were allowed to freely drink water from an automatic waterer.

Investigation items

(1) Productivity

Daily gains were obtained by measuring body weights per treatment group at the time of initiation, at week 2 and at completion of the experiment (week 6). Daily feed intake was calculated by subtracting, from an supplied amount of the feed, the remainder at the time of measuring a body weight, and feed efficiency was determined by dividing weight gain by the feeding intake. The results are shown in Table 3.

(2) Nutrient digestibility

To estimate nutrient digestibility, feed containing 0.2% of chromium oxide ($Cr_2O_3$), which was added as an indicator, was supplied to the pigs for 7 days, and then feces were collected by an anal massage method at week 2 and at completion of the experiment (week 6). The collected feces were dried in a drier at 60 °C for 72 hours and ground using a Willey mill for use in analysis. General components of the feed and Cr added as an indicator were analyzed by an AOAC (2000) method. The results are shown in Table 4.

(3) Blood profiles

For blood sampling, 8 pigs were randomly selected from each treatment group at completion of the experiment (week 6), 2 ml of blood was taken from the jugular vein using a $K_3$EDTA Vacuum tube (Becton Dicknson Vacutainer Systems, Franklin Lakes, NJ), and then white blood cells (WBCs), red blood cells (RBCs) and lymphocytes were analyzed using an automatic blood analyzer (ADVID 120, Bayer, USA). In addition, for a serum biological test, a serum obtained by taking 5 ml of blood from the jugular vein using a vacuum tube (Becton Dickinson Vacutainer Systems, Franklin Lakes, NJ) and performing centrifugation at 4 °C and 3,000 rpm for 15 minutes was analyzed by an enzymatic colorimetric method (Allain et al., 1974), and total cholesterol and HDL cholesterol were reacted with a test reagent (Cholesterol Kit No. 352, Sigma Chemical, St. Louis, MO, USA) and concentrations thereof were measured using an automatic biochemical analyzer (HITACHI 747, Japan), and an LDL cholesterol concentration was measured by a method of Naoyuki and Yoshiharu (1995). The results are shown in Table 5.

(4) Microbial composition of feces

At completion of the test (week 6), 8 pigs were selected from each treatment group, feces were collected from the pigs by an anal massage method, freeze-stored at -20 °C until use in an experiment, homogenized by suspension in sterilized saline, and then diluted serially from $10^3$ to $10^7$ folds for being used as a sample for counting viable cells. To count bacterial cells of *Lactobacillus* and *E. coli* in the porcine feces obtained by experimental treatment, *Lactobacillus* was cultured in MRS agar and *E. coli* was cultured in MacConkey agar (Difco, USA) at 37 °C for 38 hours, and then cells were counted. The results are shown in Table 6.

(5) Generation of odor substance in feces

To analyze the generation of odor in the feces of a weaning pig, contents of ammonia, total mercaptan, and hydrogen sulfide were measured. To this end, at completion of the test (week 6), feces generated by each treatment group for the same length of time were collected from all weaning pigs. 300 g of feces were collected, put into a 2,600 mL sealed plastic container, fermented for 24 hours and stored at room temperature for 5 days, and ammonia, total mercaptan, hydrogen sulfide and volatile fatty acids were measured using Gastec gas detection tubes (Model GV-100, Gastec, Japan) on day 5 of storage. The results are shown in Table 7.

(6) Fecal scores

Fecal scores were recorded daily for an entire test period and rated as follows: (Score = 1 hard, dry pellet; 2 firm, formed stool; 3 soft, moist stool that retains shape; 4 soft, unformed stool that assumes shape of container; 5 watery liquid that can be poured). The results are shown in Table 8.

Results

(1) Productivity

Treatment groups of Experimental Group 2 and Experimental Group 3 exhibited significantly higher daily gains during phase 1 than the negative control and treatment groups of comparative groups. Experimental Group 1, Experimental Group 2, Experimental Group 3, Comparative Group 2 and Comparative Group 3 exhibited significantly higher daily gains in phase 2 than the negative control. The treatment group of Experimental Group 3 exhibited significantly higher daily gains in the entire test period than those of the negative control and Comparative Group 1, and the treatment groups of Experimental Group 1 and Experimental Group 3 exhibited significantly higher feed efficiency than that of the negative control.

[Table 3]

| Investigation Items | Positive Control | Experimental Group 1 | Experimental Group 2 | Experimental Group 3 | Negative Contro l | Comparative Group 1 | Comparative Group 2 | Comparative Group 3 |
|---|---|---|---|---|---|---|---|---|
| Phase 1 (days 1 to 14) | | | | | | | | |
| Daily Gain (ADG, g) | 366 | 371 | 383 | 390 | 333 | 343 | 362 | 373 |
| Feed Intake (ADFI, g) | 424 | 421 | 428 | 430 | 397 | 387 | 401 | 418 |
| G/F | 0.863 | 0.881 | 0.895 | 0.907 | 0.839 | 0.886 | 0.903 | 0.892 |
| Phase 2 (days 14 to 42) | | | | | | | | |
| Daily Gain (ADG, g) | 521 | 537 | 543 | 553 | 492 | 519 | 537 | 541 |
| Feed Intake (ADFI, g) | 826 | 817 | 838 | 847 | 821 | 832 | 585 | 841 |
| G/F | 0.631 | 0.657 | 0.648 | 0.653 | 0.602 | 0.624 | 0.626 | 0.643 |
| (Days 1 to 42) | | | | | | | | |
| Daily Gain (ADG, g) | 469 | 482 | 490 | 499 | 439 | 460 | 479 | 485 |
| Feed Intake (ADFI, g) | 692 | 685 | 701 | 708 | 680 | 683 | 705 | 700 |
| G/F | 0.678 | 0.704 | 0.699 | 0.705 | 0.649 | 0.673 | 0.679 | 0.693 |

(2) Nutrient digestibility

As shown in Table 4 below, there was no significant difference among treatment groups in terms of dry matter, nitrogen and energy digestibility.

[Table 4]

| Investigation Items (%) | Positive Control | Experimental Group 1 | Experimental Group 2 | Experimental Group 3 | Negative Control | Comparative Group 1 | Comparative Group 2 | Comparative Group 3 |
|---|---|---|---|---|---|---|---|---|
| Week 2 | | | | | | | | |
| Dry matter | 79.60 | 80.04 | 80.68 | 79.54 | 80.23 | 79.96 | 79.27 | 80.10 |
| Nitrogen | 75.56 | 73.37 | 74.08 | 75.27 | 75.67 | 73.89 | 74.36 | 73.98 |
| Energy digestibility | 79.75 | 77.45 | 79.38 | 77.97 | 77.93 | 79.53 | 78.83 | 78.02 |
| Week 6 | | | | | | | | |
| Dry matter | 77.74 | 77.68 | 77.34 | 77.03 | 76.88 | 76.94 | 76.79 | 76.35 |
| Nitrogen | 73.00 | 72.68 | 73.23 | 73.50 | 73.40 | 72.88 | 73.03 | 72.60 |
| Energy digestibility | 76.61 | 75.70 | 75.94 | 76.05 | 76.01 | 76.40 | 76.02 | 75.98 |

(3) Blood characteristic

As shown in Table 5 below, there was no significant difference among treatment groups in terms of concentrations of WBC, RBC, lymphocytes, HDL cholesterol, LDL cholesterol and total cholesterol.

[Table 5]

| Blood Components | Positive Control | Experimental Group 1 | Experimental Group 2 | Experimental Group 3 | Negative Control | Comparative Group 1 | Comparative Group 2 | Comparative Group 3 |
|---|---|---|---|---|---|---|---|---|
| WBC, $10^3$/l | 13.46 | 15.30 | 12.73 | 15.34 | 15.87 | 14.55 | 14.61 | 15.42 |
| RBC, $10^6$/l | 5.22 | 5.30 | 5.40 | 5.75 | 5.61 | 5.77 | 5.32 | 6.08 |
| Lymphocytes ,% | 48.7 | 50.8 | 48.1 | 51.9 | 48.6 | 53.7 | 49.1 | 51.5 |
| HDL cholesterol, mg/dL | 35 | 35 | 38 | 38 | 32 | 34 | 33 | 36 |
| LDL cholesterol, mg/dL | 48 | 56 | 58 | 58 | 55 | 59 | 55 | 55 |
| Total cholesterol, mg/dL | 96 | 101 | 108 | 108 | 94 | 96 | 107 | 103 |

As shown in Table 6 below, there was no significant difference among treatment groups in terms of *Lactobacillus* and *E. coli* cell counts in feces.

[Table 6]

| Microbes (log$_{10}$cfu/g) | Positive Control | Experimental Group 1 | Experimental Group 2 | Experimental Group 3 | Negative Control | Comparative Group 1 | Comparative Group 2 | Comparative Group 3 |
|---|---|---|---|---|---|---|---|---|
| *Lactobacillus* | 7.36 | 7.40 | 7.51 | 7.53 | 7.59 | 7.42 | 7.23 | 7.49 |
| *E. coli* | 5.18 | 4.84 | 5.05 | 4.97 | 5.18 | 5.01 | 4.82 | 4.77 |

(5) Generation of odor substance in feces

As shown in Table 7 below, there was no significant difference among treatment groups in terms of generation of ammonia, hydrogen sulfate and total mercaptan.

[Table 7]

| Substances (ppm) | Positive Control | Experimental Group 1 | Experimental Group 2 | Experimental Group 3 | Negative Control | Comparative Group 1 | Comparative Group 2 | Comparative Group 3 |
|---|---|---|---|---|---|---|---|---|
| Ammonia | 11.48 | 12.10 | 11.15 | 13.10 | 10.23 | 9.58 | 9.33 | 9.68 |
| Hydrogen sulfate | 9.7 | 9.8 | 9.4 | 10.0 | 9.4 | 8.9 | 8.4 | 8.9 |
| Total mercaptan | 7.8 | 8.0 | 7.7 | 8.7 | 7.7 | 7.5 | 7.4 | 7.7 |

(6) Fecal scores

As shown in Table 8 below, there were no significant differences between treatment group in fecal scores during the entire test period.

[Table 8]

| Time of measurement | Positive Control | Experimental Group 1 | Experimental Group 2 | Experimental Group 3 | Negative Control | Comparative Group 1 | Comparative Group 2 | Comparative Group 3 |
|---|---|---|---|---|---|---|---|---|
| Week 2 | 3.0 | 3.0 | 3.0 | 3.0 | 3.0 | 3.0 | 3.0 | 3.0 |
| Week 6 | 3.0 | 3.0 | 3.0 | 3.0 | 3.0 | 3.0 | 3.0 | 3.0 |

**Experimental Example 2: Evaluation of lipid composition for broiler chicken 1**

Experimental animals and experimental design

Five hundred and ten one-day-old ROSS 308 chicks (females and males) were tested, a body weight at the initiation of the test was 41 ±0.17g, and the feeding experiment was performed for 5 weeks. Each animal group was treated as follows, each treatment was repeated six times, and 17 ROSS 308 chicks were completely randomly allotted for each treatment.

- Positive Control: Feed
- Negative Control: 100 kcal-reduced feed
- Experimental Group 1: 100 kcal-reduced feed+0.075 wt% of lipid composition of Example 1
- Experimental Group 2: 100 kcal-reduced feed+0.10 wt% of lipid composition of Example 1
- Experimental Group 3: 100 kcal-reduced feed+0.15 wt% of lipid composition of Example 1

Experimental feed, feeding and management

As experimental feed, a powder-type feed consisting mainly of corn and soybean meal and formulated in accordance with the NRC (1994) requirements was provided. ROSS 308 chicks were raised in 3-level cages, positions per treatment group were adjusted, and the feed and water were freely allowed to have.

Investigation items and methods

(1) Productivity

Weight gain was determined by measuring body weights per treatment group at the initiation, week 1, week 3 and completion (week 5) of the test. Feed intake was calculated by subtracting, from an supplied amount of the feed, the remainder at the time of measuring a body weight, and a feed conversion ratio was determined by dividing the feed intake by the weight gain.

(2) Meat quality characteristic

At completion of the test (week 5), 6 chickens were randomly selected from each treatment group and sacrificed by cervical dislocation, and then their liver, spleen, bursa of Fabricius, abdominal fat, chest muscles and gizzard were weighed and ratios thereof in the living body were calculated. pH was measured using a pH meter (77P, Istek, Korea), a meat color of each breast meat sample was measured twice using a color difference meter (Model CR-410. Minolta Co., Japan). Here, the specification of a color standard plate was L*=89.2, a*=0.921, b*=0.783. Drip loss was obtained by trimming a sample into a regular shape with a thickness of 4 cm, putting it into a polyethylene bag, and measuring losses generated on days 1, 3, 5, and 7 while storing the sample in a 4 °C refrigerator for 7 days.

(3) Blood characteristic

For blood sampling, 6 chickens were randomly selected from each type of treatment, and at completion of the test (week 5), 2 ml of blood was taken from the jugular vein using a K3EDTA Vacuum tube (Becton Dicknson Vacutainer Systems, Franklin Lakes, NJ) and analyzed, and then free fatty acids (FFAs) were investigated using an automatic blood analyzer (ADVID 120, Bayer, USA). In addition, for a serum biochemical test, at completion of the test (week 5), 5 ml of blood was taken from the jugular vein using a vacuum tube (Becton Dickinson Vacutainer Systems, Franklin Lakes, NJ) and centrifuged at 4 °C and 3,000 rpm for 15 minutes, thereby obtaining a serum, which was used in analysis. The contents of a protein, BUN, creatinine, triglyceride and glucose in the isolated serum were determined using a nephelometer (Behring, Germany) by a nephelometer method.

(4) Degree of odor generation in feces

A degree of gas generation in feces was evaluated by measuring the generation of ammonia, hydrogen sulfate and total mercaptan. To this end, at completion of the test (week 5), feces produced by each treatment group for the same length of time were collected for use in analysis. The measurement of ammonia, hydrogen sulfate and total mercaptan was carried out by putting 300 g of feces into a 2,600 mL sealed plastic container, fermenting the feces for 24 hours, storing to container at room temperature for 5 days, and then performing measurement using a Gastec gas detection tube (Model GV-100, Gastec, Japan) on day 5 of storage.

(5) Nutrient digestibility

To estimate nutrient digestibility, feed containing 0.2% of chromium oxide ($Cr_2O_3$), which was added as an indicator, was supplied to the chicks for 7 days, and then feces were collected at the completion of the experiment (week 5). The collected feces were dried in a drier at 60 °C for 72 hours and ground using a Willey mill for use in analysis. General components of the feed and Cr added as an indicator were analyzed by an AOAC (2000) method

Results

(1) Productivity

Effects of supplementation of the lipid composition of the present invention to a broiler chicken feed on productivity of the broiler chicken are shown in Table 9 below. On days 7 to 21, Experimental Group 1, Experimental Group 2 and Experimental Group 3 exhibited significantly higher body weight gains (BWGs) than the negative control, and as more lipid composition was supplemented, the body weight gain was linearly increased. Experimental Group 1, Experimental Group 2 and Experimental Group 3 exhibited significantly lower feed conversion ratios (FCRs) than the negative control, and as more lipid composition was supplemented, the feed conversion ratio was linearly decreased. On days 21 to 35, Experimental Group 1 exhibited a significantly lower feed conversion ratio than the negative control. During the entire test period, Experimental Group 1 exhibited a significantly higher body weight gain than the negative control, and as more lipid composition was supplemented, the body weight gain was linearly increased. Experimental Group 1 exhibited a significantly lower feed conversion ratio than the negative control, and as more lipid composition was supplemented, the feed conversion ratio was linearly decreased.

[Table 9]

| Investigation Items | Positive Control | Negative Control | Experimental Group | | |
|---|---|---|---|---|---|
| | | | Experimental Group 1 | Experimental Group2 | Experimental Group3 |
| Days 1 to 7 | | | | | |
| BWG, g | 189 | 187 | 191 | 192 | 193 |
| FI, g | 216 | 213 | 215 | 215 | 216 |
| FCR | 1.144 | 1.142 | 1.128 | 1.124 | 1.116 |
| Days 7 to 21 | | | | | |
| BWG, g | 574 | 557 | 591 | 600 | 600 |
| FI, g | 935 | 953 | 938 | 934 | 928 |
| FCR | 1.633 | 1.713 | 1.591 | 1.558 | 1.546 |
| Days 21 to 35 | | | | | |
| BWG, g | 906 | 893 | 917 | 918 | 963 |
| FI, g | 1535 | 1537 | 1542 | 1539 | 1540 |
| FCR | 1.699 | 1.723 | 1.686 | 1.678 | 1.608 |
| Total period | | | | | |
| BWG, g | 1668 | 1638 | 1699 | 1710 | 1756 |
| FI, g | 2686 | 2704 | 2695 | 2689 | 2683 |
| FCR | 1.612 | 1.652 | 1.588 | 1.573 | 1.531 |

(2) Carcass and meat quality characteristics

Effects of supplementation of the lipid composition of the present invention to a broiler chicken feed on carcass and meat quality characteristics of a broiler chicken are shown in Table 10 below. On day 1, the negative control exhibited a significantly higher drip loss than Experimental Group 2, and as more lipid composition was supplemented, the drip loss was linearly decreased. On day 5, the negative control exhibited a significantly higher drip loss than Experimental Group 3. On day 7, there was no significant difference in drip loss among treatment groups, but as more lipid composition was supplemented, the drip loss was linearly increased. In terms of carcass characteristics, there was no significant differences among treatment groups in terms of pH, a meat color, the liver, the spleen, the bursa of Fabricius, the abdominal fat, chest muscles and the gizzard.

[Table 10]

| Items | Positive Control | Negative Control | Experimental Group | | |
|---|---|---|---|---|---|
| | | | Experimental Group 1 | Experimental Group 2 | Experimental Group 3 |
| pH | 5.41 | 5.41 | 5.41 | 5.44 | 5.40 |
| Breast meat color | | | | | |
| Lightness (L*) | 57.14 | 57.30 | 57.43 | 57.06 | 57.38 |
| Redness (a*) | 13.77 | 13.23 | 13.35 | 13.13 | 13.36 |
| Yellowness (b*) | 12.27 | 12.39 | 12.22 | 12.42 | 23.44 |
| Drip Loss, % | | | | | |
| 1d | 2.60 | 2.76 | 1.89 | 1.70 | 1.75 |
| 3d | 5.43 | 5.43 | 5.21 | 5.05 | 5.18 |
| 5d | 8.99 | 9.93 | 8.76 | 8.60 | 8.27 |
| 7d | 14.64 | 14.46 | 13.04 | 12.98 | 12.43 |
| Relative Organ Weight, % | | | | | |
| Liver | 2.63 | 2.64 | 2.64 | 2.67 | 2.65 |
| Spleen | 0.13 | 0.13 | 0.13 | 0.13 | 0.12 |
| Bursa of Fabricius | 0.20 | 0.19 | 0.19 | 0.20 | 0.19 |
| Breast Meat | 9.49 | 9.35 | 9.50 | 9.84 | 9.78 |

(3) Blood characteristic

Effects of supplementation of the lipid composition of the present invention to a broiler chicken feed on the blood characteristics of a broiler chicken are shown in Table 11 below. There was no significant difference among treatment groups in terms of a protein, BUN, creatinine, triglyceride, FFA and glucose.

[Table 11]

| Blood Components | Positive Control | Negative Control | Experimental Group | | |
|---|---|---|---|---|---|
| | | | Experimental Group 1 | Experimental Group 2 | Experimental Group 3 |
| Protein, g/dL | 3.37 | 3.18 | 3.12 | 3.28 | 3.47 |
| BUN, mg/dL | 2.40 | 2.62 | 2.62 | 2.55 | 2.65 |
| Creatinine, mg/dL | 0.113 | 0.108 | 0.122 | 0.108 | 0.132 |
| Triglyceride, mg/dL | 48 | 45 | 43 | 41 | 43 |
| FFA, uEq/L | 449 | 443 | 434 | 443 | 460 |
| Glucose, mg/dL | 178 | 175 | 173 | 172 | 176 |

(4) Odor substance in feces

Effects of supplementation of the lipid composition of the present invention to a broiler chicken feed on the odor substance in feces of a broiler chicken are shown in Table 12 below. There was no significant difference among treatment groups in terms of ammonia, hydrogen sulfate and total mercaptan generated in feces.

[Table 12]

| Division (ppm) | Positive Control | Negative Control | Experimental Group | | |
|---|---|---|---|---|---|
| | | | Experimental Group 1 | Experimental Group 2 | Experimental Group 3 |
| $NH_3$ | 5.33 | 5.50 | 5.17 | 5.50 | 5.33 |
| R.SH | 2.42 | 2.08 | 2.25 | 2.25 | 2.08 |
| $H_2S$ | 1.75 | 1.58 | 1.58 | 1.67 | 1.25 |

(5) Nutrient digestibility

Effects of supplementation of the lipid composition of the present invention to a broiler chicken feed on the nutrient digestibility of a broiler chicken are shown in Table 13 below. Experimental Group 3 exhibited a significantly higher dry matter digestibility than the negative control, and as more lipid composition was supplemented, the dry matter digestibility was linearly increased. Experimental Group 1, Experimental Group 2 and Experimental Group 3 exhibited a significantly higher nitrogen digestibility than the negative control and the positive control, and as more lipid composition was supplemented, the nitrogen digestibility was linearly increased. Experimental Group 2 and Experimental Group 3 exhibited significantly higher energy digestibility than the negative control, and as more lipid composition was supplemented, the energy digestibility was linearly increased.

[Table 13]

| Investigation Items (%) | Positive Control | Negative Control | Experimental Group | | |
|---|---|---|---|---|---|
| | | | Experimental Group 1 | Experimental Group 2 | Experimental Group 3 |
| Dry matter digestibility | 72.56 | 71.37 | 73.17 | 73.55 | 73.75 |
| Nitrogen digestibility | 66.65 | 65.69 | 67.78 | 68.29 | 68.73 |
| Energy digestibility | 74.32 | 73.43 | 75.18 | 75.56 | 75.98 |

### Experimental Example 3: Evaluation of lipid composition for broiler chicken 2

Six hundred one-day-old Ross 308 chicks (4 treatments×5 repetitions, 30 chicks for each treatment) were fed the lipid composition of Example 1. Here, the lipid composition was added to a commercially-available broiler chicken feed at ratios of 0.1, 0.2 and 0.4 wt%, and the chicks were fed the resulting feed for 36 days. The former feed (ME, 3080 kcal/kg; CP, 20.5%) was provided for 20 days, and the latter feed (ME, 3100 kcal/kg; CP, 19.5%) was provided for 16 days.

Here, each animal group was treated as follows: (% is wt%)

- Positive Control: feed
- Experimental Group 1: feed+0.1 wt% of lipid composition of Example 1
- Experimental Group 2: feed+0.2 wt% of lipid composition of Example 1
- Experimental Group 3: feed+0.4 wt% of lipid composition of Example 1

[Table 14]

| Investigation Items | Positive Control | Experimental Group 1 | Experimental Group 2 | Experimental Group 3 |
|---|---|---|---|---|
| Initial body weight (g) | 41.5 | 41.7 | 41.6 | 41.7 |
| Final body weight (g) | 2205.4 | 2218.4 | 2293.1 | 2254.5 |
| Daily gain (g/day) | | | | |
| Days 1 to 20 | 43.8 | 44.3 | 46.1 | 46.8 |
| Days 21 to 36 | 88.8 | 89.1 | 91.6 | 87.9 |
| Days 1 to 36 | 61.8 | 62.2 | 64.3 | 63.2 |
| FI g/day/number of chickens | | | | |
| Days 1 to 20 | 68.1 | 66.8 | 67.9 | 69.4 |
| Days 21 to 36 | 173.9 | 176.9 | 185.1 | 175.4 |
| Days 1 to 36 | 110.4 | 110.8 | 114.8 | 111.8 |
| Feed Conversion Ratio (FCR) | | | | |
| Days 1 to 20 | 1.56 | 1.51 | 1.47 | 1.48 |
| Days 21 to 36 | 1.96 | 1.99 | 2.02 | 1.99 |
| Days 1 to 36 | 1.79 | 1.78 | 1.79 | 1.77 |
| Fatty acids ($\mu$Eq/L) | 317.7 | 211.50 | 387.7 | 411.6 |
| Triglyceride (mg/dL) | 92.60 | 99.20 | 56.60 | 63.00 |

As shown in Table 14, a tendency of increased body weight with the supplementation of the lipid composition of Example 1 was observed. In the early stage of the experiment, daily body weight gain increased significantly and linearly with the addition of the lipid composition, and the feed conversion ratio was also linearly improved.

A blood free fatty acid concentration was linearly increased by addition of the lipid composition of Example 1, and in contrast, a blood triglyceride concentration was linearly and significantly decreased.

**Experimental Example 4: Evaluation of lipid composition for flatfish 1**

Forty flatfish per water tank were raised, and the average body weight of the fish in each water tank was 13.4 g. A sufficient amount of feed was provided at 8:00 and 18:00. A temperature of the water tank was 15 to 21 °C, and the fish were raised for 12 weeks. Here, each animal group was treated (fed) as shown in Table 15 below. To investigate a growth rate and a feed utilization efficiency, a specific growth rate (%), a feed intake, a feed conversion ratio, and a protein efficiency ratio were calculated as follows, and the results are shown in FIGS. 1 to 4.

$$\text{Specific growth rate } (\%) = (\text{Ln final weight (g)} - \text{Ln initial weight (g)})/\text{experimental days} \times 100$$

Feed intake (g/fish) = dry feed fed/fish
Feed conversion ratio = dry feed fed/wet weight gain
Protein efficiency ratio = wet weight gain/total protein intake

$$\text{Survival rate } (\%) = \text{final fish number/initial fish number} \times 100$$

[Table 15]

| Ingredient (wt%) | Control | Experimental Group |
|---|---|---|
| Fish powder | 55.0 | 55.0 |
| Soybean meal | 6.0 | 6.0 |
| Corn gluten meal | 4.0 | 4.0 |
| Flour | 26.5 | 26.5 |
| Mixed minerals | 1.0 | 1.0 |
| Mixed vitamins | 1.0 | 1.0 |
| Choline chloride | 0.5 | 0.5 |
| Fish oil | 6.0 | 3.0 |
| Fat composition of Example 1 | - | 3.0 |

FIG. 1 is a set of graphs representing a body weight and body weight gain of flatfish measured at completion of an experiment, FIG. 2 is a set of graphs representing a specific growth rate and a feed intake of flatfish, FIG. 3 is a set of graphs representing a feed conversion ratio and a protein efficiency ratio, and FIG. 4 is a graph representing a survival rate of flatfish.

As shown in FIGS. 1 to 4, as a result of raising flatfish with the feed from which 50 wt% of a lipid source of a flatfish feed was substituted with the lipid composition of the present invention, there was no significant difference between the control and the experimental groups in terms of the body weight, specific growth rate (%) and survival rate of flatfish measured at completion of the experiment.

**Experimental Example 5: Evaluation of lipid composition for flatfish 2**

Fifty flatfish per isolated water tank were raised, and the average body weight of the fish in each water tank was 6.8 g. A sufficient amount of feed was provided at 8:00 and 18:00. A temperature of the water tank was 23 to 28 °C, and the fish were raised for 8 weeks. Here, each animal group was fed as shown in Table 16 below. The results are shown in FIGS. 5 to 8.

[Table 16]

| Ingredient (wt%) | Control | Experimental Group |
|---|---|---|
| Fish powder | 55.0 | 55.0 |
| Soybean meal | 6.0 | 6.0 |
| Corn gluten meal | 4.0 | 4.0 |
| Flour | 26.5 | 26.5 |
| Mixed minerals | 1.0 | 1.0 |
| Mixed vitamins | 1.0 | 1.0 |
| Choline chloride | 0.5 | 0.5 |
| Fish oil | 6.0 | 4.0 |
| Starch | - | 1.5 |
| Fat composition of Example 1 | - | 0.5 |

FIG. 5 is a set of graphs representing a body weight and body weight gain of flatfish measured at completion of the experiment, FIG. 6 is a set of graphs representing a specific growth rate and a feed intake of flatfish, FIG. 7 is a set of graphs representing a feed conversion ratio and a protein efficiency ratio, and FIG. 8 is a graph representing a survival rate of flatfish.

As shown in FIGS. 5 to 8, it can be confirmed that the final body weight of flatfish was increased by the supplementation of the lipid composition of Example 1.

**Claims**

1. A weight gain promoting feed additive, which is a lipid composition comprising, for a total of 100 wt%:

    50 to 70 wt% of a diglyceride; and
    a triglyceride, a monoglyceride, free fatty acids or a mixture thereof as the remainder,
    wherein the diglyceride comprises 40 wt% or more of 1,3-a diglyceride,
    among constitutive fatty acids of the diglyceride, fatty acids having 14 carbon atoms or less account for 70 to 90 wt% and fatty acids having 16 carbon atoms or more account for 10 to 30 wt%, and
    fatty acids having 14 carbon atoms or less account for 60 to 80 wt% with respect to fatty acids bound to the 1 and 3 positions among the constitutive fatty acids of the diglyceride.

2. The weight gain promoting feed additive of claim 1, wherein, in the constitutive fatty acids, a weight ratio of the fatty acids having 14 carbon atoms or less over the fatty acids having 16 carbon atoms or more is 4 to 7.

3. The weight gain promoting feed additive of claim 1, wherein the fatty acid having 14 carbon atoms or less is selected from the group consisting of caprylic acid, pelargonic acid, capric acid, undecanoic acid, lauric acid, myristic acid and a mixture thereof.

4. The weight gain promoting feed additive of claim 1, wherein the fatty acid having 16 carbon atoms or more is selected from the group consisting of palmitic acid, oleic acid, linoleic acid, stearic acid and a mixture thereof.

5. A livestock feed composition, comprising:

    the weight gain promoting feed additive according to any one of claims 1 to 4; and
    a formulated feed mixture.

6. The feed composition of claim 5, which is used for raising pigs, chickens, ducks, quails, goose, pheasants, turkeys, cow, bulls, cows, horses, donkeys, sheep, goat, dogs, cats, rabbits, or raised fish or shrimps.

7. The feed composition of claim 5, wherein the weight gain promoting feed additive is contained at 0.01 to 5.0 wt% based on the total weight of the formulated feed mixture.

8. A livestock breeding method, comprising:
    providing the livestock feed composition of claim 5.

9. A livestock feed composition, in which 40 to 60 wt% of a lipid source of the formulated feed mixture is substituted with the weight gain promoting feed additive according to any one of claims 1 to 4.

10. The feed composition of claim 9, which is used for raising pigs, chickens, ducks, quails, goose, pheasants, turkeys, cow, bulls, cows, horses, donkeys, sheep, goat, dogs, cats, rabbits, or raised fish or shrimps.

11. A livestock breeding method, comprising:
    supplying the livestock feed composition of claim 10.

[Fig. 1]

**Final body weight**

**Weight gain**

---- Initial fish weight

[Fig. 2]

**Specific growth rate**

**Feed intake**

[Fig. 3]

**Feed conversion ratio**

**Protein efficiency ratio**

[Fig. 4]

**Survival**

[Fig. 5]

[Fig. 6]

[Fig. 7]

[Fig. 8]

Survival

## INTERNATIONAL SEARCH REPORT

International application No.

**PCT/KR2017/003077**

| A. CLASSIFICATION OF SUBJECT MATTER |
|---|
| *A23K 20/105(2016.01)i, A23K 50/00(2016.01)i* |
| According to International Patent Classification (IPC) or to both national classification and IPC |

| B. FIELDS SEARCHED |
|---|
| Minimum documentation searched (classification system followed by classification symbols) |
| A23K 20/105; A23D 7/00; C11C 3/00; A23K 1/14; A23K 1/18; A23D 9/00; C11B 3/00; A23K 1/175; A23K 1/16; A23K 50/00 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: diglyceride, triglyceride, monoglyceride, free fatty acid, feed for livestock, facilitate weight gain |

| C. DOCUMENTS CONSIDERED TO BE RELEVANT | | |
|---|---|---|
| Category* | Citation of document, with indication, where appropriate, of the relevant passages | Relevant to claim No. |
| A | JP 2007-512407 A (ONBIO CORPORATION) 17 May 2007<br>See abstract; paragraphs [0011]-[0012], [0018], [0021], [0038], [0055], [0059]; claim 1. | 1-11 |
| A | JP 05-056755 A (KAO CORPORATION) 09 March 1993<br>See abstract; paragraphs [0006], [0009]; claims 1-2. | 1-11 |
| A | KR 10-2004-0036941 A (KAO CORPORATION) 03 May 2004<br>See abstract; pages 3-4; claim 1. | 1-11 |
| A | US 5859270 A (KOLSTAD et al.) 12 January 1999<br>See the entire document. | 1-11 |
| A | KR 10-2010-0042085 A (CHONG WON PIG-RAISING AND MANAGEMENT-FARM ASSOCIATION CORPORATION) 23 April 2010<br>See the entire document. | 1-11 |
| PX | KR 10-2016-0143488 A (YUN, Kwan Sik) 14 December 2016<br>See abstract; paragraphs [0024]-[0029]. | 1-11 |

☐ Further documents are listed in the continuation of Box C.   ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>10 JULY 2017 (10.07.2017) | Date of mailing of the international search report<br><br>**10 JULY 2017 (10.07.2017)** |
| Name and mailing address of the ISA/KR<br>Korean Intellectual Property Office<br>Government Complex-Daejeon, 189 Seonsa-ro, Daejeon 302-701,<br>Republic of Korea<br>Facsimile No. +82-42-481-8578 | Authorized officer<br><br><br>Telephone No. |

Form PCT/ISA/210 (second sheet) (January 2015)

International application No.

**PCT/KR2017/003077**

| Patent document cited in search report | Publication date | Patent family member | Publication date |
|---|---|---|---|
| JP 2007-512407 A | 17/05/2007 | CN 1906280 A | 31/01/2007 |
| | | CN 1906280 B | 09/06/2010 |
| | | EP 1733012 A1 | 20/12/2006 |
| | | KR 10-0740564 B1 | 18/07/2007 |
| | | KR 10-2005-0052384 A | 02/06/2005 |
| | | US 2007-0141220 A1 | 21/06/2007 |
| | | US 2010-0280112 A1 | 04/11/2010 |
| | | WO 2005-052102 A1 | 09/06/2005 |
| JP 05-056755 A | 09/03/1993 | CA 2071345 A1 | 18/12/1992 |
| | | CA 2071345 C | 03/09/2002 |
| | | EP 0519458 A1 | 23/12/1992 |
| | | EP 0519458 B1 | 27/12/1996 |
| | | JP 3034678 B2 | 17/04/2000 |
| | | US 5462967 A | 31/10/1995 |
| KR 10-2004-0036941 A | 03/05/2004 | AU 2002-328058 B2 | 24/04/2008 |
| | | CA 2495146 A1 | 27/03/2003 |
| | | CA 2495146 C | 20/04/2010 |
| | | CN 1582116 A | 16/02/2005 |
| | | CN 1582116 C | 01/11/2006 |
| | | EP 1424907 A1 | 09/06/2004 |
| | | EP 1424907 B1 | 20/02/2008 |
| | | JP 2003-160794 A | 06/06/2003 |
| | | JP 4116844 B2 | 09/07/2008 |
| | | US 2004-0265466 A1 | 30/12/2004 |
| | | US 7182971 B2 | 27/02/2007 |
| | | WO 03-024237 A1 | 27/03/2003 |
| US 5859270 A | 12/01/1999 | AU 1997-21995 B2 | 12/10/2000 |
| | | EP 1021500 A1 | 26/07/2000 |
| | | US 2002-0128500 A1 | 12/09/2002 |
| | | US 5959128 A | 28/09/1999 |
| | | US 6153773 A | 28/11/2000 |
| | | WO 97-33955 A1 | 18/09/1997 |
| KR 10-2010-0042085 A | 23/04/2010 | KR 10-0979537 B1 | 02/09/2010 |
| KR 10-2016-0143488 A | 14/12/2016 | KR 10-1719598 B1 | 24/03/2017 |
| | | WO 2016-195369 A1 | 08/12/2016 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 200635444
- JP 2007512407 A
- JP 8269478 A
- JP 5056755 A
- KR 100740564